# EUROPEAN PATENT APPLICATION

(11) **EP 4 772 604 A1**
(43) Date of publication of application: **08.07.2026**
(21) Application number: 24847525.3
(22) Date of filing: 02.02.2024
(51) Int. Cl.: C12M 1/00, C12M 1/38, C12M 1/36, C12M 1/34, C12M 1/26, C12M 1/12, C12M 1/04, C12M 1/02

(54) **DIRECTED MICRO-ECOLOGY EXPANSION APPARATUS, EXPANSION METHOD AND COMPUTER-READABLE STORAGE MEDIUM**

(30) Priority: 31.07.2023 CN 202310943775
(71) Applicant: BEIJING GREEN NITROGEN BIOTECHNOLOGY CO., LTD., Beijing 100094 (CN); ZHONGNONG CHUANGDA (BEIJING) ENVIRONMENTAL PROTECTION TECHNOLOGY CO., LTD., Beijing 101499 (CN)
(72) Inventor: MA, Ruiqiang, Beijing 100094 (CN); CUI, Wenjing, Beijing 100094 (CN); YANG, Yang, Beijing 100094 (CN); HOU, Zhenhua, Beijing 100094 (CN); ZENG, Xianfeng, Beijing 100094 (CN)
(74) Representative: Murgitroyd & Company
(86) International application number: PCT/CN2024/075426
(87) International publication number: WO 2025/025556

(57) **Abstract**

The embodiment of the disclosure provides a directed micro-ecology fermentation device. The device may comprise: a housing; a fermentation container, which is provided in the housing and used for providing a fermentation place for the microbial inoculant; a liquid injection port, which is provided on the housing, communicated with the fermentation container, and used for injecting a first liquid into the fermentation container; a microbial inoculant adding unit, which is provided on the housing, communicated with the fermentation container and used for adding the microbial inoculant into the fermentation container; a temperature control unit, which is provided in the housing and used for controlling the temperature of the first liquid in the fermentation container; a stirrer, which is provided in the fermentation container and used for stirring the first liquid in the fermentation container to promote fermentation; and a discharging port, which is provided on the housing, communicated with the fermentation container and used for discharging fermented materials in the fermentation container. The embodiment of the disclosure provides a fermentation method for the directed micro-ecology fermentation device, a computer readable storage medium and an electronic apparatus.

## Description

### TECHNICAL FIELD

The present disclosure relates to the field of microorganisms cultivation, in particular to a micro-ecology quantitative fermentation system, and more specifically to a directed micro-ecology fermentation device, fermentation method and computer readable storage medium.

### BACKGROUND

The micro-ecology fermentation technology mainly includes the screening and cultivation of micro-ecology strains and the large-scale fermentation of micro-ecology bacteria species. The fermentation of micro-ecology bacteria species requires consideration of various growth conditions of the micro-ecology bacteria species, which mainly include temperature, pH value, oxygen content, species age, inoculation amount, or the like.

In the process of realizing the concept of the present disclosure, the inventors found that there are at least the following problems in the related art: the microorganisms fermentor system is large in scale, has high investment and operation cost, has a complex process flow, and requires professional personnel to operate. Moreover, after being produced in a centralized manner, the fermentation products need to undergo long-distance transportation to reach the end users, and the survival rate of microorganisms is not high after the products arrive at the end users, which cannot meet their needs.

Therefore, there is an urgently need in this field to develop a micro-ecology fermentation device to solve the problems of large scale of microbial inoculant products, high investment and operation cost, complex process flow, and the need for professional personnel to operate, as well as the problem of, after being produced in a centralized manner, the microbial inoculant products need to undergo long-distance transportation to reach the end users, and the survival rate of microorganisms is not high after the products arrive at the end users, which cannot meet their needs.

### SUMMARY

In view of this, the technical problem to be solved by the present disclosure is to provide a directed micro-ecology fermentation device, a fermentation method and a computer readable storage medium, which solve the problems in the related art that large scale of microbial inoculant products, high investment and operation cost, complex process flow, and the need for professional personnel to operate, as well as the problem of, after being produced in a centralized manner, the microbial inoculant products need to undergo long-distance transportation to reach the end users, and the survival rate of microorganisms is not high after the products arrive at the end users, which cannot meet their needs.

In order to solve the above technical problems, the specific embodiments of the present disclosure provide a directed micro-ecology fermentation device, which includes: a housing; a fermentation container, which is provided in the housing and configured to provide a place for fermentation of a microbial inoculant; a liquid injection port, which is provided on the housing, communicated with the fermentation container and configured to inject a first liquid into the fermentation container; a microbial inoculant adding unit, which is provided on the housing, communicated with the fermentation container, and configured to add the microbial inoculant into the fermentation container; a temperature control unit, which is provided in the housing and configured to control a temperature of the first liquid in the fermentation container; a stirrer, which is provided in the fermentation container and configured to stir the first liquid in the fermentation container to promote fermentation; and a discharge port, which is provided on the housing, communicated with the fermentation container and configured to discharge fermented materials in the culture container.

The specific embodiments of the present disclosure also provide a fermentation method for the directed micro-ecology fermentation device. The fermentation method includes: controlling an electronic valve to inject a predetermined amount of the first liquid into the fermentation container; sterilizing the injected first liquid by means of a non-complete sterilization manner; adding a microbial inoculant into the fermentation container according to a liquid level of the first liquid after non-complete sterilization; and performing, by adopting set conditions, fermentation on the first liquid to which the microbial inoculant is added according to a type of the microbial inoculant.

Another aspect of the embodiments of the present disclosure provides a computer program, the computer program includes computer executable instructions, and the instructions are used to implement the method of the embodiments of the present disclosure when executed.

Another aspect of the embodiments of the present disclosure provides a computer readable storage medium storing computer executable instructions, the instructions are used to implement the method of the embodiments of the present disclosure when executed by a processor.

Another aspect of the embodiments of the present disclosure provides an electronic apparatus, including one or more processors and a storage device, wherein the storage device is used to store executable instructions, and when the executable instructions are executed by the processor, the method of the embodiments of the present disclosure is implemented.

According to the above embodiments of the present disclosure, an integrated directed micro-ecology fermentation device can be used to solve at least a part of the problems in the related art that large scale of microbial inoculant products, high investment and operation cost, complex process flow, and the need for professional personnel to operate, as well as the problem of, after being produced in a centralized manner, the fermentation products need to undergo long-distance transportation to reach the end users, and the survival rate of microorganisms is not high after the products arrive at the end users, which cannot meet their needs. Therefore, the technical effects of low cost, one-click standardized production, no need for transportation, ready-to-use, and high survival rate of microorganisms can be achieved.

It should be understood that the above general description and the following specific embodiments are merely exemplary and illustrative and are not intended to limit the scope of the present disclosure.

### BRIEF DESCRIPTION OF THE DRAWINGS

The accompanying drawings below are part of the specification of the present disclosure, and illustrate exemplary embodiments of the present disclosure. Together with the description of the specification, the accompanying drawings are used to explain the principles of the present disclosure.
FIG. 1 is a first structural schematic diagram of a directed micro-ecology fermentation device provided in specific embodiments of the present disclosure.
FIG. 2A is a schematic diagram of the structure of a stirrer blade provided in specific embodiments of the present disclosure.
FIG. 2B is a schematic diagram of a flow direction of a first liquid when a stirrer provided by specific embodiments of the present disclosure is stirring in a fermentation container.
FIG. 3 is a second structural schematic diagram of the directed micro-ecology fermentation device provided in specific embodiments of the present disclosure.
FIG. 4 is a third structural schematic diagram of the directed micro-ecology fermentation device provided in specific embodiments of the present disclosure.
FIG. 5 is a fourth structural schematic diagram of the directed micro-ecology fermentation device provided in specific embodiments of the present disclosure.
FIG. 6 is a fifth structural schematic diagram of the directed micro-ecology fermentation device provided in specific embodiments of the present disclosure.
FIG. 7 is a structural schematic diagram of an electronic switch provided in specific embodiments of the present disclosure.
FIG. 8 is a sixth structural schematic diagram of the directed micro-ecology fermentation device provided in specific embodiments of the present disclosure.
FIG. 9 is a first schematic flow chart of a fermentation method of the directed micro-ecology fermentation device provided in specific embodiments of the present disclosure.
FIG. 10 is a second schematic flow chart of fermentation method of a directed micro-ecology fermentation device provided in specific embodiments of the present disclosure.
FIG. 11 is a third schematic flow chart of fermentation method of a directed micro-ecology fermentation device provided in specific embodiments of the present disclosure.
FIG. 12 is a fourth schematic flow chart of fermentation method of a directed micro-ecology fermentation device provided in specific embodiments of the present disclosure.
FIG. 13 is a schematic flow chart of a method for sterilizing an injected first liquid by means of a non-complete sterilization manner provided by specific embodiments of the present disclosure.
FIG. 14 is a schematic flow chart of a method of aerobic fermentation of a first liquid added with a microbial inoculant using set conditions provided by specific embodiments of the present disclosure.
FIG. 15 is a schematic flow chart of a method of anaerobic fermentation of a first liquid added with a microbial inoculant using set conditions provided by specific embodiments of the present disclosure.

**Description of reference signs:**

| | | | |
|---|---|---|---|
| 1 | housing | 2 | fermentation container |
| 3 | liquid injection port | 4 | microbial inoculant adding unit |
| 5 | temperature control unit | 6 | stirrer |
| 7 | discharge port | 8 | liquid storage tank |
| 9 | ejecting head | 10 | first drive unit |
| 11 | first electronic valve | 12 | second electronic valve |
| 13 | third electronic valve | 14 | fourth electronic valve |
| 15 | drain outlet | 16 | fifth electronic valve |
| 17 | aeration unit | 18 | air filter |
| 19 | liquid level detector | 20 | controller |
| 21 | temperature detector | 4-1 | microbial inoculant box |
| 4-2 | microbial inoculant delivery pipeline | 4-3 | electronic switch |
| 4-3-1 | rotation shaft | 4-3-2 | blade |
| 4-3-3 | third drive unit | 5-1 | liquid jacket |
| 5-2 | heater | 5-3 | cooler |
| 5-4 | second drive unit | | |

### DETAILED DESCRIPTION OF EMBODIMENTS

In order to make the purposes, technical solutions and advantages of the embodiments of the present disclosure clearer, the following will clearly illustrate the spirit of the content disclosed in the present disclosure with the drawing and detailed descriptions. Any person skilled in the art can change and modify the content of the present disclosure on the basis of the technology taught by the content of the present disclosure after understanding the embodiments of the content of the present disclosure, which does not depart from the spirit and scope of the content of the present disclosure.

The exemplary embodiments and descriptions thereof of the present disclosure are used to explain the present disclosure, but not to limit the present disclosure. In addition, elements/members with the same or similar numerals used in the drawings and the embodiments are used to represent the same or similar parts.

The terms "first", "second", ... etc. used herein do not specifically refer to a sequence or order, nor are they used to limit the present disclosure, but are only used to distinguish elements or operations described with the same technical terms.

Directional terms used herein, such as up, down, left, right, front or back, etc., are only used with reference to the directions of the drawings. Therefore, the directional terms used are intended to illustrate and not to limit the present disclosure.

As used herein, "comprising", "including", "having", "containing" and so on are all open terms, meaning including but not limited to.

As used herein, "and/or" includes any or all combinations of the stated things.

As used herein, the term "multiple" includes "two" and "more than two"; the term "multiple sets" includes "two sets" and "two or more sets".

The terms "approximately", "about", and the like used herein are used to modify any quantity or error that may vary slightly, but these slight changes or errors will not change its essence. Generally speaking, the range of slight changes or errors modified by such terms may be 20% in some embodiments, 10% in some embodiments, 5% or other numerical values in some embodiments. Those skilled in the art should understand that the aforementioned values can be adjusted according to actual needs, and are not limited thereto.

All terms (including technical and scientific terms) used herein have the same meaning as commonly understood by one of ordinary skill in the art unless otherwise defined. It should be noted that the terms used herein should be interpreted as having a meaning consistent with the context of this specification and should not be interpreted in an idealized or overly rigid manner.

When using expressions such as "at least one of A, B, and C, etc.", they should generally be interpreted according to the meaning of the expression commonly understood by those skilled in the art (for example, "a system having at least one of A, B, and C" should include but is not limited to a system having A alone, B alone, C alone, A and B, A and C, B and C, and/or A, B, C, etc.). When using expressions such as "at least one of A, B, or C", they should generally be interpreted according to the meaning of the expression commonly understood by those skilled in the art (for example, "a system having at least one of A, B or C" should include but is not limited to a system having A alone, B alone, C alone, A and B, A and C, B and C, and/or A, B, C, etc.). Those skilled in the art should also understand that any transitional conjunctions and/or phrases that essentially represent two or more optional items, whether in the specification or drawings, should be understood to give the possibility of including one of these items, either of these items, or both of these items. For example, the phrase "A or B" should be understood to include the possibilities of "A" or "B", or "A and B".

In the related art, conventional liquid fermentation equipment requires large investment and high technical requirements. Only professional personnel can operate it. In addition, in order to ensure that the product is still stable when it arrives at the customer, the fermentation broth generally needs to be processed into specific preparations. After microbial inoculants go through layers of distribution and reach the customers, not only has the activity of the microorganisms decreased significantly, but the terminal prices of the products are also very high, which makes it impossible for users to accept it, or the dosage is insufficient within an acceptable cost range, thus failing to achieve the desired effect and causing them to lose confidence in the use of microbial inoculants.

The present disclosure realizes the automation and standardized control of the fermentation of microbial inoculants, simplifies apparatus and reduces production cost through the optimization of apparatus, process and culture medium. No professional operating skills are required. One just starts the automatic control button of the apparatus to obtain fresh, high bacterial amount, low-cost micro-ecology bacterial liquid within a specific time, making the micro-ecology bacterial liquid a regular product that most users can afford and use at a low cost.

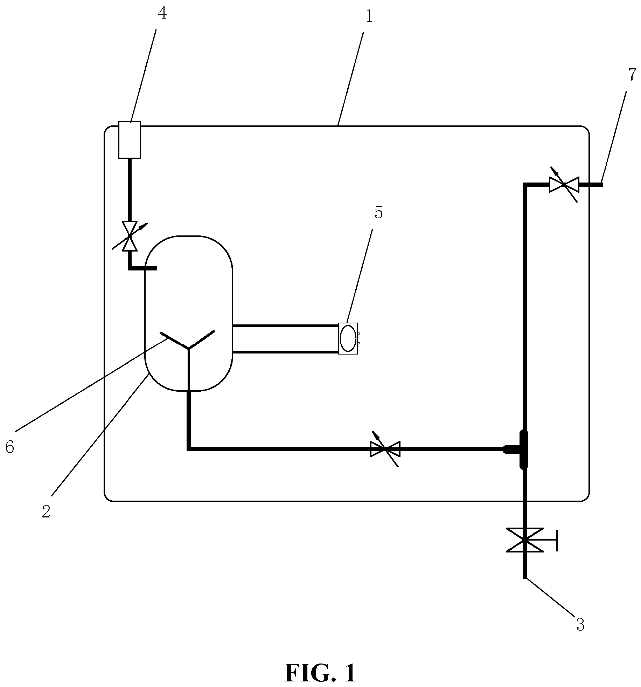

FIG. 1 is a first structural schematic diagram of a directed micro-ecology fermentation device provided in specific embodiments of the present disclosure.

As shown in FIG. 1, the directed micro-ecology fermentation device may include a housing 1, a fermentation container 2, a liquid injection port 3, a microbial inoculant adding unit 4, a temperature control unit 5, a stirrer 6 and a discharge port 7.

Specifically, the fermentation container 2 is arranged in the housing 1, and the fermentation container 2 is used to provide a place for the fermentation of microbial inoculant. The liquid injection port 3 is provided on the housing 1, and is communicated with the fermentation container 2. The liquid injection port 3 is used to inject a first liquid into the fermentation container 2. The microbial inoculant adding unit 4 is provided on the housing 1, and the microbial inoculant adding unit 4 is communicated with the fermentation container 2. The microbial inoculant adding unit 4 is used to add the microbial inoculant into the fermentation container 2. The temperature control unit 5 is provided in the housing 1, and is used to control the temperature of the first liquid in the fermentation container 2. The stirrer 6 is provided in the fermentation container 2, and the stirrer 6 is used to stir the first liquid in the fermentation container 2 to promote fermentation. The discharge port 7 is provided on the housing 1, and is communicated with the fermentation container 2. The discharge port 7 is used to discharge the fermented materials in the fermentation container 2.

In an embodiment of the present disclosure, the liquid injection port 3 may be connected to a faucet through a hose, and the faucet injects the first liquid into the fermentation container 2 through the hose. The required raw materials can be added to clean water or tap water to form the first liquid, and the first liquid may be used as a medium solution. The pH value of the first liquid may be adjusted according to the strains to be cultured to meet the needs of culturing. Clean water or tap water may also be placed in a bucket, and then the raw materials are put into the bucket to form the first liquid. The hose is placed in the bucket, and the first liquid is pumped into the fermentation container 2. The working place of the directed micro-ecology fermentation device is not limited. Alternatively, clean water or tap water may be directly injected into the fermentation container 2, and the raw materials may be added from the top of the fermentation container 2. The liquid injection port 3 may also be provided with a manual switch, as shown in the drawings. The first liquid can be injected into the fermentation container 2 only when the manual switch is turned on. Setting the manual switch can prevent users from misoperation.

In the embodiments of the present disclosure, one-touch automation and standardized control of micro-ecology fermentation are realized, the apparatus is simple, does not require transportation, and is ready-to-use, the micro-ecology survival rate is high, the production cost is low, and no professional operating skills are required. Only an automatic control button of the apparatus needs to be started to obtain fresh, high bacterial amount, low cost micro-ecology bacterial liquid within a specific time. The directed micro-ecology fermentation device is small in size, light in weight, and occupies little space. The entire fermentation process can be completed in less than 24 hours. The entire device is only the size of a washing machine, and users can transport it at will using ordinary vehicles.

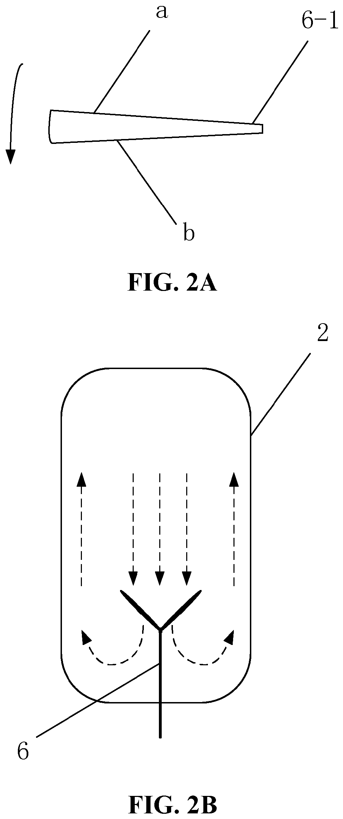

FIG. 2A is a schematic diagram of the structure of a stirrer blade provided in specific embodiments of the present disclosure. FIG. 2B is a schematic diagram of the flow direction of the first liquid when the stirrer provided by specific embodiments of the present disclosure is stirring in the fermentation container.

As shown in FIGs. 2A and 2B, the blade 6-1 of the stirrer 6 is provided at an angle, and the blade 6-1 of the stirrer 6 rotates in the direction indicated by the arrow in the drawing. The front side b of the blade 6-1 is tilted, and the rear side a of the blade 6-1 is drooped. That is, the distance between the front side b and the bottom of the fermentation container 2 is greater than the distance between the rear side a and the bottom of the fermentation container 2. The blade 6-1 of the stirrer 6 rotates, and the first liquid in the fermentation container 2 flows in the direction shown in FIG. 2B. The stirrer 6 can fully stir the first liquid in the fermentation container 2.

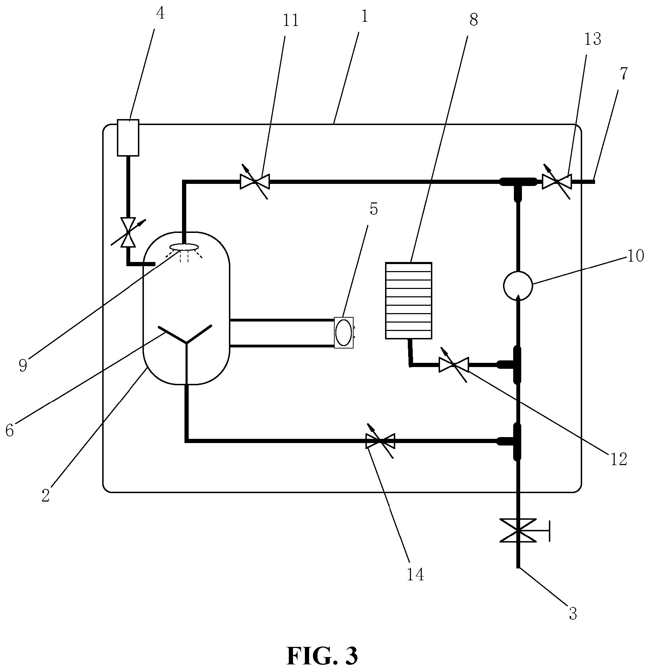

FIG. 3 is a second structural schematic diagram of the directed micro-ecology fermentation device provided in specific embodiments of the present disclosure.

As shown in FIG. 3, the directed micro-ecology fermentation device may further include a liquid storage tank 8, a ejecting head 9 and a first driving unit 10.

Specifically, the liquid storage tank 8 is provided in the housing 1, and is communicated with the liquid injection port 3 and the fermentation container 2. The liquid storage tank 8 is used to store the first liquid used for fermentation and/or the liquid for cleaning the residue on the inner wall of the fermentation container 2. The ejecting head 9 is provided inside the fermentation container 2, and is communicated with the liquid injection port 3 and the liquid storage tank 8. The ejecting head 9 is used to clean the residue on the inner wall of the fermentation container 2. The first driving unit 10 is provided between the ejecting head 9 and the liquid storage tank 8, and is used to drive the liquid in the liquid storage tank 8 to clean the residue on the inner wall of the fermentation container 2. The first driving unit 10 is also used to drive the fermented materials in the fermentation container 2 to be discharged from the discharge port 7.

In the embodiments of the present disclosure, the liquid in the liquid storage tank 8 for cleaning the residue on the inner wall of the fermentation container 2 may be the first liquid, or may be clean water or tap water. The liquid storage tank 8 can only store liquid for cleaning the residue on the inner wall of the fermentation container 2. When using the liquid in the liquid storage tank 8 to clean the residue on the inner wall of the fermentation container 2, the water supply is sufficient, and the residue on the inner wall of the fermentation container 2 can be quickly washed and cleaned thoroughly. The liquid storage tank 8 can separately store the first liquid used for fermentation and the liquid for cleaning the residue on the inner wall of the fermentation container 2. For example, the liquid storage tank 8 contains two cavities, one cavity is used to store the first liquid used for fermentation, and the other cavity is used to store the liquid for cleaning the residue on the inner wall of the fermentation container 2. The first driving unit 10 drives the fermented materials in the fermentation container 2 to be discharged from the discharge port 7, and the materials that cannot be discharged remains on the inner wall of the fermentation container 2, so the residue on the inner wall of the fermentation container 2 is mainly the residual material.

In the embodiments of the present disclosure, the liquid storage tank 8 may be a water tank. The ejecting head 9 may be a shower head. The first driving unit 10 may be a water pump.

In the embodiments of the present disclosure, the liquid storage tank 8 stores liquid, and the liquid stored in the liquid storage tank 8 is used to clean the liquid remaining on the inner wall of the fermentation container 2. The liquid storage tank 8 supplies sufficient liquid, rendering the residue on the inner wall of the fermentation container 2 can be quickly washed and cleaned thoroughly.

In the embodiments of the present disclosure, the directed micro-ecology fermentation device may further include: a first electronic valve 11, a second electronic valve 12, a third electronic valve 13 and a fourth electronic valve 14.

Specifically, the first electronic valve 11 is provided between the ejecting head 9 and the liquid injection port 3. The second electronic valve 12 is provided between the liquid storage tank 8 and the liquid injection port 3. Here, the first electronic valve 11 and the second electronic valve 12 jointly control the first liquid in the liquid storage tank 8 to clean the residue on the inner wall of the fermentation container 2 through the ejecting head 9. The third electronic valve 13 is provided between the discharge port 7 and the liquid injection port 3. The fourth electronic valve 14 is provided between a bottom opening 2-1 of the fermentation container 2 and the liquid injection port 3. Here, the third electronic valve 13 and the fourth electronic valve 14 jointly control the fermented materials in the fermentation container 2 to be discharged through the discharge port 7, or jointly control the residue after cleaning to be discharged through the discharge port 7.

In the embodiments of the present disclosure, when the second electronic valve 12 is opened, the first liquid and/or the liquid for cleaning the residue on the inner wall of the fermentation container 2 is injected into the liquid storage tank 8 through the liquid injection port 3. When the first electronic valve 11 and the second electronic valve 12 are opened, the first driving unit 10 is started, so that the liquid in the liquid storage tank 8 can be used to clean the residue on the inner wall of the fermentation container 2. When the third electronic valve 13 and the fourth electronic valve 14 are opened and the first driving unit 10 is started, the fermented materials in the fermentation container 2 can be discharged through the discharge port 7, and the residue after clearing in the fermentation container 2 can also be discharged through the discharge port 7.

In the embodiments of the present disclosure, the injecting of the first liquid into the fermentation container 2 and the discharging of the fermented materials in the fermentation container 2 share some pipelines, thereby raw materials for producing the directed micro-ecology fermentation device are saved and the space occupied by the directed micro-ecology fermentation device is reduced. The cleaning of the residue on the inner wall of the fermentation container 2, the discharging of the fermented materials in the fermentation container 2 and the discharging of the residue after clearing in the fermentation container 2 share some pipelines, thereby raw materials for producing the directed micro-ecology fermentation device are further saved and the space occupied by the directed micro-ecology fermentation device is further reduced.

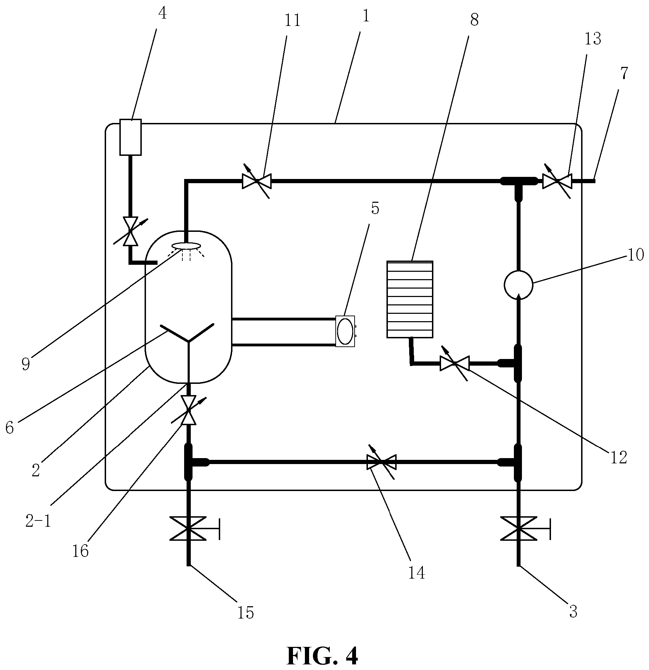

FIG. 4 is a third structural schematic diagram of the directed micro-ecology fermentation device provided in specific embodiments of the present disclosure.

As shown in FIG. 4, the directed micro-ecology fermentation device may further include a drain outlet 15 and a fifth electronic valve 16.

Specifically, the drain outlet 15 is communicated with the bottom opening 2-1 through a pipe, and the drain outlet 15 is provided on the housing 1. The drain outlet 15 is used to discharge the residue after cleaning. The fifth electronic valve 16 is provided between the bottom opening 2-1 and the drain port 15, and is used to control the discharge of the residue after cleaning through the drain port 15.

In the embodiments of the present disclosure, when the fourth electronic valve 14 is closed and the fifth electronic valve 16 is opened, the residue after cleaning is discharged through the drain port 15. After discharging the fermented materials in the fermentation container 2, the residue in the fermentation container 2 is directly cleaned and discharged through the drain outlet 15 to prevent the residue in the fermentation container 2 from affecting the next fermentation, which is convenient for continued use next time. It is one-click operated, does not require professional operating skills, and is safe and reliable. The drain outlet 15 may also be provided with a manual switch, as shown in the drawing, and the residue can be discharged through the drain outlet 15 only when the manual switch is turned on. Setting the manual switch can prevent users from misoperation.

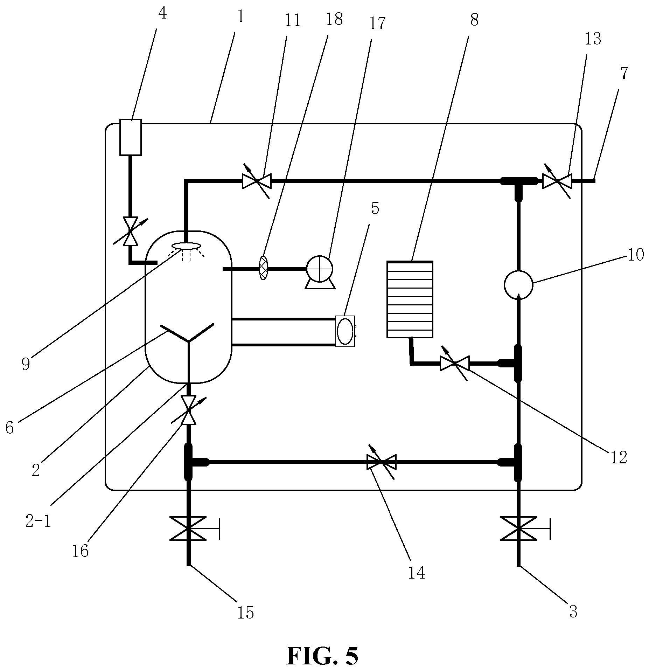

FIG. 5 is a fourth structural schematic diagram of the directed micro-ecology fermentation device provided in specific embodiments of the present disclosure.

As shown in FIG. 5, the directed micro-ecology fermentation device may further include an aeration unit 17 and an air filter 18.

Specifically, the aeration unit 17 is provided in the housing 1, and the aeration unit 17 is communicated with the fermentation container 2. The aeration unit 17 is used to introduce air into the fermentation container 2 so that the aerobic fermentation for the microbial inoculant can be performed. The air filter 18 is provided between the aeration unit 17 and the fermentation container 2, and is used to filter impurities and moisture in the air.

In the embodiments of the present disclosure, the aeration unit 17 may include a blower or the like. The air filter 18 may include a filter membrane or the like. The aeration unit 17 directly introduces air into the fermentation container 2. The air source is wide, and the working place of the directed micro-ecology fermentation device is not limited. The air filter 18 filters out impurities and moisture in the air to prevent the impurities and moisture in the air from affecting the effect of the fermentation. The directed micro-ecology fermentation device is provided with the aeration unit 17, which can be suitable for the fermentation of aerobic strains and the fermentation of anaerobic strains, as well as the enrichment of microbial metabolites, thereby further expanding the application scope of the directed micro-ecology fermentation device.

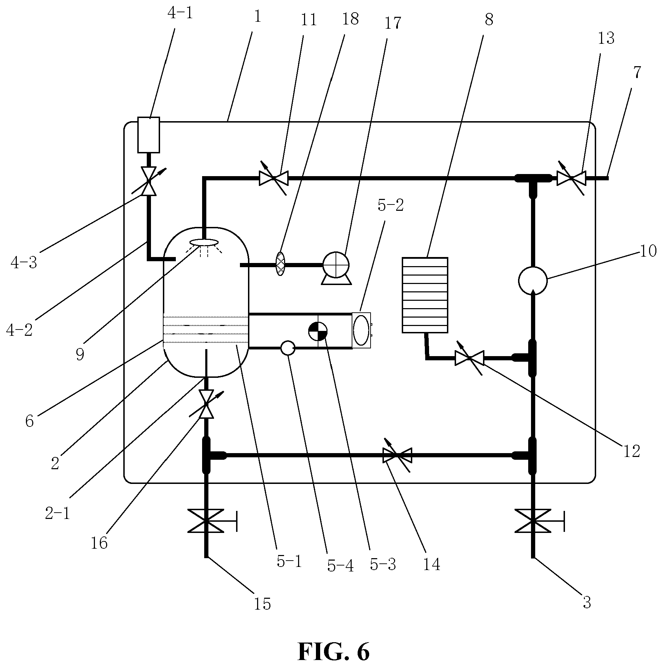

FIG. 6 is a fifth structural schematic diagram of the directed micro-ecology fermentation device provided in specific embodiments of the present disclosure.

As shown in FIG. 6, the temperature control unit 5 may include a liquid jacket 5-1, a heater 5-2, a cooler 5-3 and a second driving unit 5-4.

The liquid jacket 5-1 is provided inside the shell of the fermentation container 2, and the liquid jacket 5-1 is used to heat or cool the first liquid in the fermentation container 2 by using the second liquid in the liquid jacket 5-1 by means of heat transfer, wherein the second liquid in the liquid jacket 5-1 is isolated from the first liquid in the fermentation container 2. The heater 5-2 is used to heat the second liquid in the liquid jacket 5-1, wherein a liquid outlet of the heater 5-2 is communicated with the liquid inlet of the liquid jacket 5-1, and the liquid inlet of the heater 5-2 is communicated with the liquid outlet of the liquid jacket 5-1. The cooler 5-3 is communicated in parallel with the heater 5-2, and the cooler 5-3 is used to cool the second liquid in the liquid jacket 5-1. The second driving unit 5-4 is communicated in series with the heater 5-2, and the second driving unit 5-4 is used to drive the second liquid in the liquid jacket 5-1 to circulate.

In the embodiments of the present disclosure, the second liquid may include at least one of oil, water, and liquid gas. The heater 5-2 may include an electromagnetic heating system or the like. The cooler 5-3 may include a tube type cooler, a plate cooler, an air-cooled cooler, and the like. If the second liquid is oil, the heater 5-2 can heat the oil to above 100°C to pasteurize the first liquid (culture medium) in the fermentation container 2. Then, the cooler 5-3 cools the first liquid to below 40°C so that the microbial inoculant can be added for fermentation.

In the embodiments of the present disclosure, the microbial inoculant adding unit 4 may include a microbial inoculant box 4-1, a microbial inoculant delivery pipeline 4-2 and an electronic switch 4-3.

Specifically, the microbial inoculant box 4-1 is provided in the housing 1, and the microbial inoculant box 4-1 is used to store the microbial inoculants. The microbial inoculant delivery pipeline 4-2 is provided between the microbial inoculant box 4-1 and the fermentation container 2, and is used to deliver the microbial inoculants stored in the microbial inoculant box 4-1 to the fermentation container 2, wherein the microbial inoculant delivery pipeline 4-2 is made of heat-insulating material. The electronic switch 4-3 is provided between the microbial inoculant box 4-1 and the microbial inoculant delivery pipeline 4-2. The electronic switch 4-3 is used to deliver a predetermined amount of the microbial inoculants to the fermentation container 2 through the microbial inoculant delivery pipeline 4-2 under the control of a signal.

In the embodiments of the present disclosure, the microbial inoculant delivery pipeline 4-2 is made of a heat-insulating material. After the fermentation container 2 is heated by the liquid jacket 5-1, the heat of the fermentation container 2 will not be transferred to the microbial inoculant box 4-1, which can ensure that the microbial inoculant box 4-1 is always in a low temperature state and will not affect the activity of the microbial inoculants in the microbial inoculant box 4-1. The microbial inoculant delivery pipeline 4-2 may be made of wooden material or heat-insulating polymer material, but the present disclosure is not limited thereto.

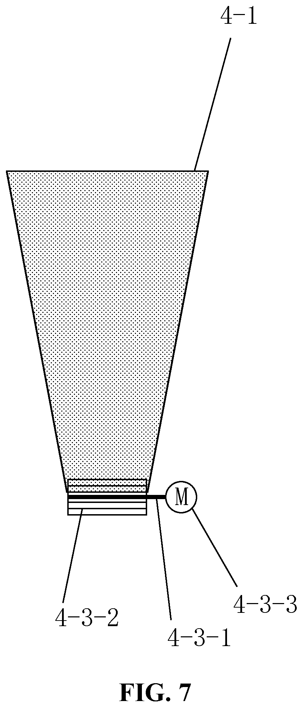

FIG. 7 is a structural schematic diagram of the electronic switch provided in specific embodiment of the present disclosure.

As shown in FIG. 7, the microbial inoculant box 4-1 is conical, and the electronic switch 4-3 may include a rotation shaft 4-3-1, a plurality of blades 4-3-2 and a third driving unit 4-3-3.

Specifically, the rotation shaft 4-3-1 is provided at the bottom of the microbial inoculant box 4-1. A plurality of blades 4-3-2 are evenly provided around the rotation shaft 4-3-1, wherein the rotation shaft 4-3-1 and the blades 4-3-2 prevent the microbial inoculants in the microbial inoculant box 4-1 from entering the microbial inoculant delivery pipeline 4-2. The third driving unit 4-3-3 is connected to the rotation shaft 4-3-1, and the third driving unit 4-3-3 is used to drive the rotation shaft 4-3-1 to rotate, thereby driving the blades 4-3-2 to rotate, so as to intake the microbial inoculants in the microbial inoculant box 4-1 into the microbial inoculant delivery pipeline 4-2.

In the embodiments of the present disclosure, the rotation angle of the rotation shaft 4-3-1 is proportional to the amount of the microbial inoculants intook into the microbial inoculant delivery pipeline 4-2. Therefore, the amount of the microbial inoculants intook into the microbial inoculant delivery pipeline 4-2 can be controlled by controlling the rotation angle of the rotation shaft 4-3-1. The present disclosure can accurately control the amount of microbial inoculants added by controlling the rotation angle of the rotation shaft 4-3-1.

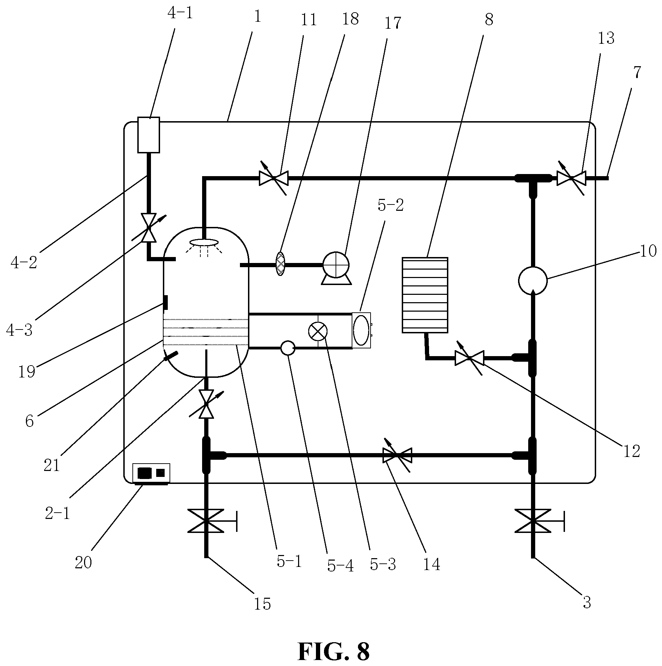

FIG. 8 is a sixth structural schematic diagram of the directed micro-ecology fermentation device provided in specific embodiments of the present disclosure.

As shown in FIG. 8, the directed micro-ecology fermentation device may further include a liquid level detector 19, a controller 20 and a temperature detector 21.

Specifically, the liquid level detector 19 is provided in the fermentation container 2, and the liquid level detector 19 is used to monitor the liquid level of the first liquid in the fermentation container 2. The controller 20 is electrically connected to the liquid level detector 19 and the third driving unit 4-3-3, and the controller 20 is used to control the rotation angle of the rotation shaft 4-3-1 according to the liquid level of the first liquid. The temperature detector 21 is provided in the fermentation container 2. The temperature detector 21 is electrically connected to the controller 20, the heater 5-2, the cooler 5-3 and the second driving unit 5-4 respectively. The temperature detector 21 is used to monitor the temperature of the first liquid in the fermentation container 2. Here, the controller 20 controls the heater 5-2, the cooler 5-3 and the second driving unit 5-4 to operate according to the temperature of the first liquid in the fermentation container 2.

In the embodiments of the present disclosure, the controller 20 controls the rotation angle of the rotation shaft 4-3-1 according to the liquid level of the first liquid, thereby controlling the amount of the microbial inoculants intook into the microbial inoculant delivery pipeline 4-2, and accurately controlling the amount of the microbial inoculants added. When the first liquid in the fermentation container 2 needs to be pasteurized, the heater 5-2 and the second driving unit 5-4 work, the heater 5-2 heats the second liquid in the liquid jacket 5-1, and the second liquid heats the first liquid in the fermentation container 2. When the first liquid in the fermentation container 2 needs to be cooled, the cooler 5-3 and the second driving unit 5-4 work, the cooler 5-3 cools the second liquid in the liquid jacket 5-1, and the second liquid cools the first liquid in the fermentation container 2.

In the embodiments of the present disclosure, the directed micro-ecology fermentation device may further include a display screen and a control key (or a touch screen). The user may set the fermentation amount (half-fermentor fermentation or full-frementor fermentation) through the control key, may choose aerobic strain fermentation or anaerobic strain fermentation, or even choose a specific fermentation strain. The user may also choose whether to store the materials in the fermentation container 2 or discharge them through the discharge port 7 after the fermentation is completed. After discharging the materials through the discharge port 7, the device can be set to automatically clean the fermentation container 2 by means of one-key control, and no professional operating skills are required.

The present disclosure realizes the automation and standardized control of the microbial inoculant fermentation, has simple apparatus and low production cost. No professional operating skills are required. One just starts the automatic control button of the apparatus to obtain fresh, high bacterial amount, low-cost micro-ecology bacterial liquid within a specific time.

The most widely used micro-ecology strains at present include Bacillus subtilis, Bacillus licheniformis, Bacillus amyloliquefaciens, Lactobacillus plantarum, Lactobacillus casei, Rhizobium, etc., which can basically be divided into aerobic strains and anaerobic strains. The aerobic strains include Bacillus subtilis, Bacillus licheniformis, Bacillus amyloliquefaciens, and the like. The anaerobic strains include Lactobacillus plantarum, Lactobacillus casei, Rhizobium, and the like. Below are examples of aerobic micro-ecology strain fermentation and anaerobic micro-ecology strain fermentation, as well as examples of polyglutamic acid and rhamnolipids micro-ecology strain fermentation and enrichment, respectively.

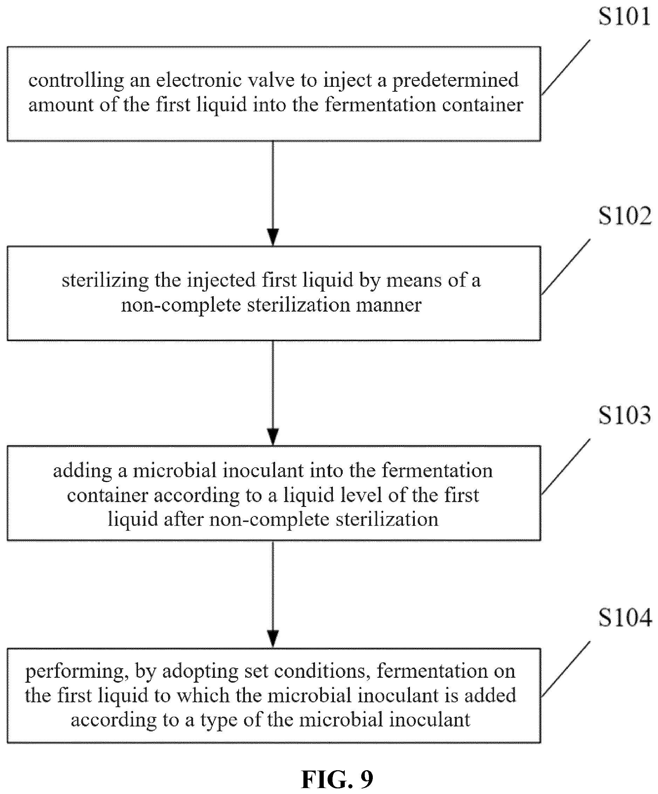

FIG. 9 is a first schematic flow chart of a fermentation method of the directed micro-ecology fermentation device provided in specific embodiments of the present disclosure.

As shown in FIG. 9, the fermentation method of the directed micro-ecology fermentation device may include the following operations S101~S104:

In operation S101: controlling the electronic valve to inject a predetermined amount of the first liquid into the fermentation container.

In the embodiments of the present disclosure, the first liquid may be a medium solution, and the medium may be added into clean water or tap water to form the culture medium liquid. The liquid level of the first liquid in the fermentation container 2 can be monitored by the liquid level detector 19.

Then, in operation S102: sterilizing the injected first liquid by means of a non-complete sterilization manner.

In the embodiments of the present disclosure, after the first liquid is sterilized by means of a non-complete sterilization manner, theoretically, both target bacteria and miscellaneous bacteria exist in the first liquid. The target bacteria can only begin to proliferate rapidly after a certain lag period. Sterilizing the first liquid by means of a non-complete sterilization manner can effectively suppress the rapid proliferation of miscellaneous bacteria and effectively reduce the base number of miscellaneous bacteria in the medium, thereby providing favorable conditions for the rapid proliferation of target bacteria.

Next, in operation S103: adding a microbial inoculant into the fermentation container according to the liquid level of the first liquid after non-complete sterilization.

In the embodiments of the present disclosure, the user can arbitrarily set the amount of the first liquid injected into the fermentation container 2 as needed, and the amount of the first liquid injected into the fermentation container 2 is reflected by the liquid level of the first liquid. The larger the amount of the first liquid, the more microbial inoculants are added, and the user can set the amount of fermentation as needed.

Then, in operation S104: performing, by adopting set conditions, fermentation on the first liquid to which the microbial inoculant is added according to the type of the microbial inoculant.

In the embodiments of the present disclosure, the microbial inoculants may include the anaerobic microbial inoculants and the aerobic microbial inoculants. The aerobic microbial inoculants may include Bacillus subtilis, Bacillus licheniformis, Bacillus amyloliquefaciens, and the like. The anaerobic microbial inoculants may include Lactobacillus plantarum, Lactobacillus casei, Rhizobium, and the like. The set conditions may include fermentation temperature, stirring speed, ventilation volume, fermentation time, and the like.

In the embodiments of the present disclosure, one-touch automation and standardized control of microbial inoculant fermentation are realized, the apparatus is simple, does not require transportation, and is ready-to-use, the micro-ecology survival rate is high, the production cost is low, and no professional operating skills are required. Only an automatic control button of the apparatus needs to be started to obtain fresh, high bacterial amount, low cost micro-ecology bacterial liquid within a specific time. The entire fermentation process can be completed in less than 24 hours, with high fermentation efficiency.

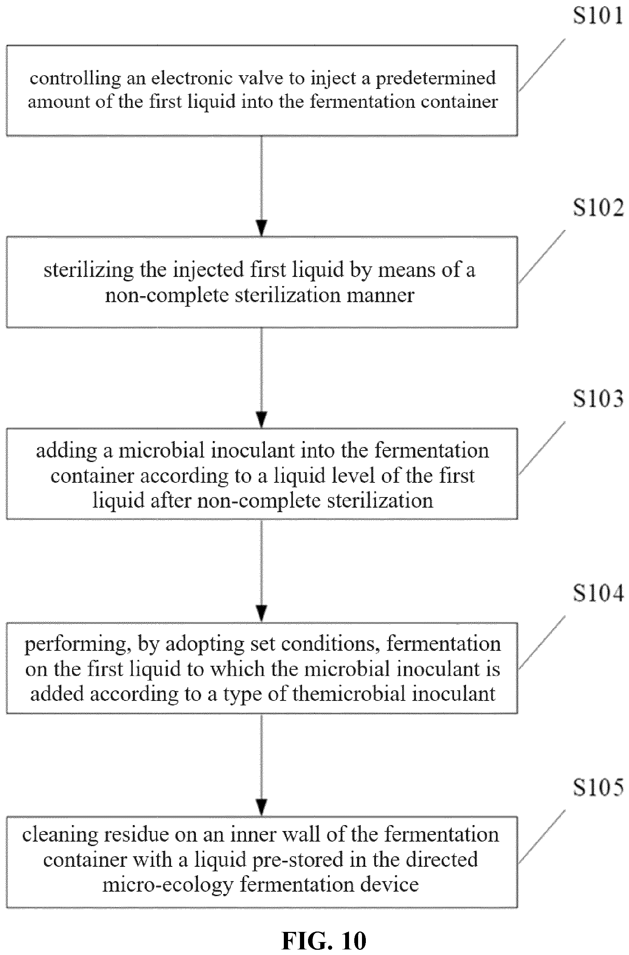

FIG. 10 is a second schematic flow chart of a fermentation method of the directed micro-ecology fermentation device provided in specific embodiments of the present disclosure.

As shown in FIG. 10, after the operation S104, i.e., performing, by adopting set conditions, fermentation on the first liquid to which the microbial inoculant is added according to the type of the microbial inoculant, the fermentation method of the directed micro-ecology fermentation device may further include the following operation S105:

In operation S105: cleaning the residue on the inner wall of the fermentation container with the liquid pre-stored in the directed micro-ecology fermentation device.

In the embodiments of the present disclosure, the liquid used for cleaning the residue can be stored in the liquid storage tank 8 built in the directed micro-ecology fermentation device, and the liquid used for cleaning the residue may include water or a special liquid. The liquid used for cleaning the residue is stored in the liquid storage tank 8, and a large amount of liquid can be used for flushing to achieve thorough cleaning.

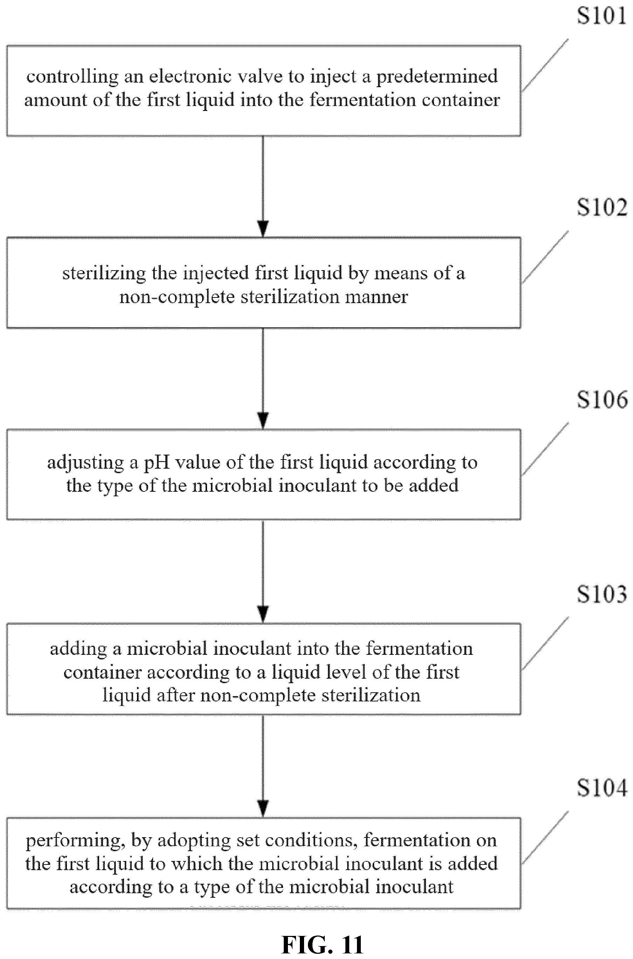

FIG. 11 is a third schematic flow chart of a fermentation method of the directed micro-ecology fermentation device provided in specific embodiments of the present disclosure.

As shown in FIG. 11, before the operation S103, i.e., adding a microbial inoculant into the fermentation container according to the liquid level of the first liquid after non-complete sterilization, the fermentation method of the directed micro-ecology fermentation device may further include the following operation S106:

In operation S106: adjusting the pH value of the first liquid according to the type of the microbial inoculant to be added.

In the embodiments of the present disclosure, the initial pH value of the first liquid directly affects the pH value of the first liquid after the non-complete sterilization, thereby affecting the fermentation effect. Experiments have shown that for aerobic microbial inoculants, the initial pH value of the first liquid can be adjusted to 7.6~7.8 to achieve the maximum expansion effect. For anaerobic microbial inoculants, the initial pH value of the first liquid can be adjusted to 6.8~7.0.

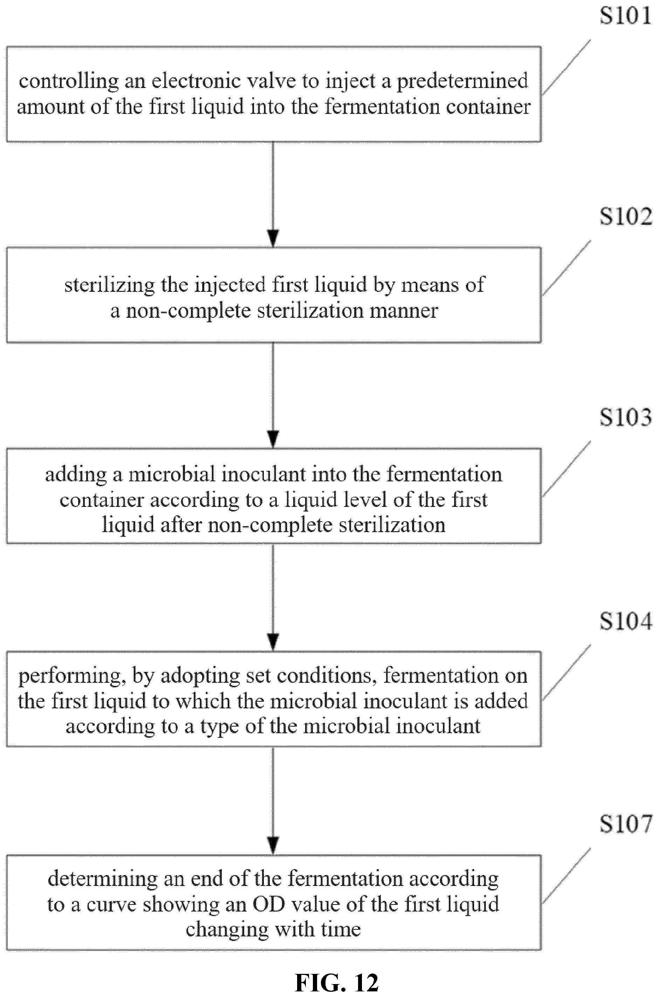

FIG. 12 is a fourth schematic flow chart of a fermentation method of the directed micro-ecology fermentation device provided in specific embodiments of the present disclosure.

As shown in FIG. 12, after the operation S104, i.e., performing, by adopting set conditions, fermentation on the first liquid to which the microbial inoculant is added according to the type of the microbial inoculant, the fermentation method of the directed micro-ecology fermentation device may further include the following operation S107:

In operation S107: determining the end of the fermentation according to a curve showing the OD value of the first liquid changing with time.

In the embodiments of the present disclosure, the OD value can reflect the content of the target bacteria in the first liquid. The larger the OD value, the higher the content of the target bacteria in the first liquid. As the fermentation time increases, the OD value increases. After a certain period of time (for example, 12~18 hours), the OD value reaches a peak value, and then the OD value begins to decrease. The time when the OD value reaches the peak value is the end of the fermentation.

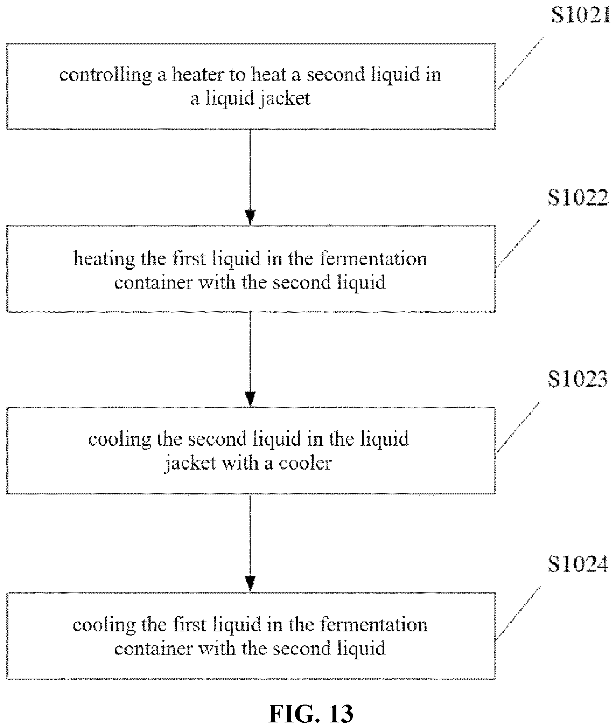

FIG. 13 is a schematic flow chart of a method for sterilizing the injected first liquid by means of the non-complete sterilization manner provided by specific embodiments of the present disclosure.

As shown in FIG. 13, the operation S102, i.e., sterilizing the injected first liquid by means of a non-complete sterilization manner, may include the following operations S1021~S1024:

Controlling the heater to heat a second liquid in the liquid jacket, wherein the liquid jacket is provided inside the shell of the fermentation container, and the second liquid is isolated from the first liquid.

In the embodiments of the present disclosure, the second liquid may include at least one of oil, water and liquid gas. If the second liquid is oil, the boiling point of the oil can exceed 100°C, and the temperature of the first liquid can be heated to 100°C, which can eliminate more miscellaneous bacteria in the first liquid and lay a good foundation for the efficient fermentation of the microbial inoculants in the first liquid.

Heating the first liquid in the fermentation container with the second liquid.

In the embodiment of the present disclosure, the second liquid is isolated from the first liquid, and the second liquid heats the first liquid in the fermentation container by heat transfer.

Cooling the second liquid in the liquid jacket with the cooler.

In the embodiments of the present disclosure, the cooler 5-3 may not be started, thus the second liquid in the liquid jacket is allowed to automatically cool to room temperature, but the cooling process is relatively long.

Cooling the first liquid in the fermentation container with the second liquid.

In the embodiments of the present disclosure, the second liquid is isolated from the first liquid, and the first liquid transfers heat to the second liquid by heat transfer.

In the embodiments of the present disclosure, the heater 5-2 and the cooler 5-3 cooperate to sterilize the second liquid and promote efficient fermentation of the microbial inoculants in the first liquid, thereby achieving efficient utilization of the temperature control unit 5.

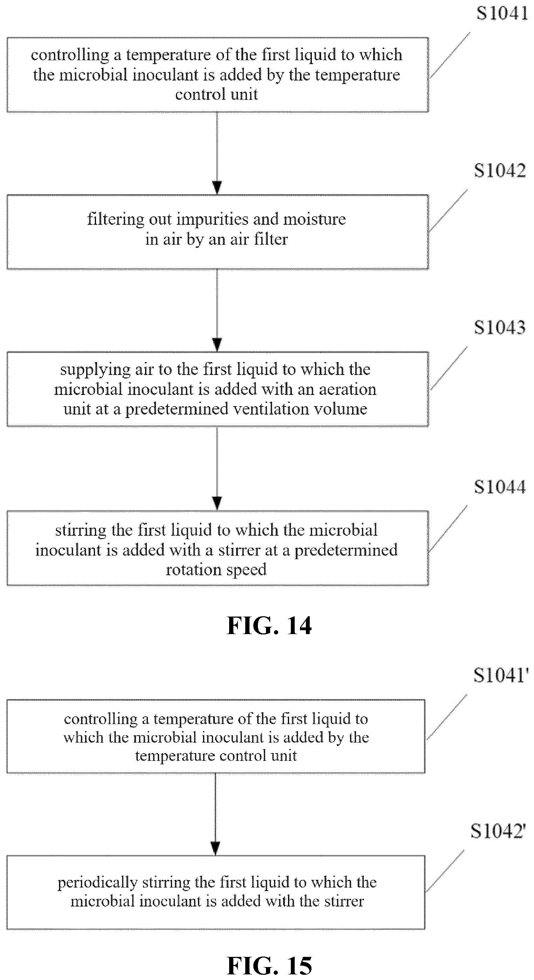

FIG. 14 is a schematic flow chart of a method of aerobic fermentation of the first liquid added with the microbial inoculant using set conditions provided by specific embodiments of the present disclosure.

As shown in FIG. 14, if the microbial inoculant is an aerobic microbial inoculant, the operation S104, i.e., performing, by adopting set conditions, fermentation on the first liquid to which the microbial inoculant is added according to the type of the microbial inoculant, may include the following operations S1041~S1044:

In operation S1041: controlling the temperature of the first liquid to which the microbial inoculant is added by the temperature control unit.

In the embodiments of the present disclosure, experiments have shown that the temperature control unit can be used to keep the first liquid at a constant temperature (for example, 30~40°C) to achieve stability in the fermentation of aerobic bacteria species. Thus the fermentation is not easily affected by the environment and can be carried out in fields, woods, waterside and other places, with a wide range of applications.

In operation S1042: filtering out impurities and moisture in the air by the air filter.

In the embodiments of the present disclosure, the air filter can be a filter membrane. Filtering out impurities and moisture in the air can prevent miscellaneous bacteria in the air from entering the first liquid, allowing the fermentation of the target bacteria stable.

In operation S1043: supplying air to the first liquid to which the microbial inoculant is added with an aeration unit at a predetermined ventilation volume.

In the embodiments of the present disclosure, experiments have shown that a ventilation volume of 2-3 vvm is sufficient for the fermentation of the aerobic bacteria.

In operation S1044: stirring the first liquid to which the microbial inoculant is added with a stirrer at a predetermined rotation speed.

In the embodiments of the present disclosure, experiments have shown that stirring the first liquid to which the microbial inoculant is added at a rotation speed of 200-300 rpm can satisfy the fermentation of the aerobic bacteria.

In the embodiments of the present disclosure, the operation S1043, i.e., supplying air to the first liquid to which the microbial inoculant is added with an aeration unit at a predetermined ventilation volume, may include the following operation: supplying air to the first liquid to which the microbial inoculant is added with an aeration unit at a ventilation volume of 2-3 vvm. The operation S1044, i.e., stirring the first liquid to which the microbial inoculant is added by a stirrer at a predetermined rotation speed, may include the following operation: stirring the first liquid to which the microbial inoculant is added by a stirrer at a rotation speed of 200-300 rpm.

FIG. 15 a schematic flow chart of a method of anaerobic fermentation of the first liquid added with the microbial inoculant using set conditions provided by specific embodiments of the present disclosure.

As shown in FIG. 15, if the microbial inoculant is an anaerobic microbial inoculant, the operation S104, i.e., performing, by adopting set conditions, fermentation on the first liquid to which the microbial inoculant is added according to the type of the microbial inoculant, may include the following operations S1041'~S1042':

In operation S1041': controlling the temperature of the first liquid to which the microbial inoculant is added with the temperature control unit.

In the embodiments of the present disclosure, experiments have shown that the temperature control unit can be used to keep the first liquid at a constant temperature (for example, 33~37°C) to achieve stability in the fermentation of anaerobic bacteria species. Thus the fermentation is not easily affected by the environment and can be carried out in fields, woods, waterside and other places, with a wide range of applications.

In operation S1042': periodically stirring the first liquid to which the microbial inoculant is added with the stirrer.

In the embodiments of the present disclosure, experiments have shown that stirring the first liquid for one minute every three hours can achieve efficient fermentation of the microbial inoculant in the first liquid.

In the embodiments of the present disclosure, the operation S1042', i.e., periodically stirring the first liquid to which the microbial inoculant is added with the stirrer, may include the following operation: stirring the first liquid for one minute every three hours with the stirrer.

In the embodiments of the present disclosure, users can choose the fermentation of aerobic bacteria inoculants or the fermentation of anaerobic bacteria inoculants or the enrichment of microbial metabolites according to their needs. According to the type of microbial inoculant, fermentation parameters such as fermentation temperature, stirring speed and ventilation volume are automatically set. It can be completed with one click, and the operation is simple and clear. No professional guidance is required. Users can operate it by themselves. It can be used as soon as it is produced, no transportation is required, the survival rate of microorganisms is high, and the production cost is low. It is suitable for wide application in agriculture, forestry, animal husbandry and fishery and other fields.

### Example of aerobic micro-ecology strain fermentation

### 1. Determining the optimal pH value of the medium

One can turn on the manual switch of the liquid injection port 3 and the fourth electronic valve 14 of the directed micro-ecology fermentation device, and inject clean water or tap water into the fermentation container 2, or directly inject the first liquid into the fermentation container 2. If clean water or tap water is injected into the fermentation container 2, the medium can be added through the top opening of the fermentation container 2 to form the first liquid (medium). The pH value of the first liquid is to be tested, and the pH value of the aerobic micro-ecology system is alkaline. The initial pH value of the first liquid directly affects the pH value of the medium after pasteurization, and the pH value has a direct impact on the fermentation of the strains. Based on the relatively mature fermentation medium, Bacillus subtilis is used as the representative strain for aerobic fermentation. The selection of the most suitable initial pH values derived experimentally is shown in Table 1 below. Table 1 shows the effect of the initial pH values of aerobic micro-ecology strain fermentation on the amount of bacteria in fermentation:

**Table 1**

| fermentation mode | initial pH value | pH value after sterilization | amount of bacteria |
|---|---|---|---|
| | 7.80 | 7.35 | 6.27 billion |
| | 7.60 | 7.17 | 5.98 billion |
| aerobic fermentation | 7.40 | 7.06 | 5.53 billion |
| | 7.20 | 6.98 | 4.84 billion |
| | 7.00 | 6.73 | 2.29 billion |
| | 6.80 | 6.55 | 1.86 billion |

Conclusion: taking Bacillus subtilis as an example, the most suitable initial pH value for aerobic strain fermentation is 7.6~7.8.

### 2. Determining the optimal sterilization temperature

The second liquid is heated by the heater 5-2, and the second liquid heats the first liquid (medium) in the fermentation container 2, and the medium is pasteurized (heating power 5~7kw). Under a stirring condition, the medium is gradually heated from room temperature to 90~100°C, and the heating time is about 2~3h; then the second liquid is cooled by the cooler 5-3, and the second liquid cools the first liquid in the fermentation container 2, and the temperature is gradually reduced to 30~40°C, and the cooling time is about 1.5~3h (the specific cooling time is related to the room temperature). The microbial inoculant (i.e., Bacillus subtilis species) is added to the cooled fermentation container 2 through the microbial inoculant adding unit 4.

Pasteurization treatment is a non-complete sterilization system. Theoretically, both target bacteria and miscellaneous bacteria exist in the medium. After the target bacteria species enter the fermentation container 2, it will take a certain lag period for them to begin to proliferate rapidly. Therefore, it is necessary to effectively suppress the rapid proliferation of the miscellaneous bacteria in the early stage of culture. Pasteurization treatment can effectively reduce the base number of the miscellaneous bacteria in the medium. It is simple to operate and economical, thus providing favorable conditions for the rapid proliferation of target bacteria.

Referring to the tracking results of the changes in the amount of aerobic fermentation bacteria and OD value with the fermentation time (see Table 7), after the target bacteria being inoculated, the OD value of the fermentation solution begins to increase rapidly after 4 hours, and the strain proliferation enters the logarithmic phase after 8 hours. Therefore, in order to ensure that the target bacteria are the dominant bacteria group before the end of the fermentation, it is necessary to control the rapid proliferation of miscellaneous bacteria within 8 hours of blank culture.

To simulate pasteurization conditions, same mediums (LB medium) were treated at different temperatures for a certain period of time, and then cultured at 37°C with sterile air (without adding microbial inoculants). The changes in the OD value (absorbance value) of the treated miscellaneous bacteria were tracked and detected. The results are shown in Table 2 below. Table 2 shows the effect of pasteurization conditions on the proliferation of miscellaneous bacteria during the fermentation of the aerobic micro-ecology strains:

**Table 2**

| processing temperature | processing time | 0h | 2h | 4h | 6h | 8h |
|---|---|---|---|---|---|---|
| 60 | 0 | 0.0726 | 0.092 | 0.537 | 1.244 | 2.485 |
| | 60min | 0.078 | 0.087 | 0.126 | 0.979 | 1.815 |
| | 90min | 0.08 | 0.097 | 0.282 | 0.811 | 1.13 |
| | 120min | 0.079 | 0.098 | 0.306 | 0.961 | 1.9 |
| 70 | 0 | 0.073 | 0.112 | 0.105 | 0.643 | 1.72 |
| | 60min | 0.076 | 0.091 | 0.09 | 0.162 | 1.362 |
| | 90miin | 0.081 | 0.093 | 0.206 | 1.15 | 2.795 |
| | 120min | 0.078 | 0.089 | 0.1 | 0.528 | 1.435 |
| 80 | 0 | 0.0776 | 0.082 | 0.089 | 0.305 | 1.016 |
| | 10min | 0.0736 | 0.101 | 0.092 | 0.323 | 1.93 |
| | 30min | 0.0779 | 0.081 | 0.088 | 0.213 | 0.775 |
| | 60min | 0.078 | 0.097 | 0.088 | 0.095 | 0.599 |
| 90 | 0 | 0.068 | 0.086 | 0.087 | 0.151 | 0.660 |
| | 10min | 0.069 | 0.083 | 0.089 | 0.080 | 0.103 |
| | 30min | 0.072 | 0.086 | 0.082 | 0.099 | 0.306 |
| | 60min | 0.079 | 0.087 | 0.093 | 0.089 | 0.117 |

Conclusion: after pasteurization, within 8 hours of culture, the changes in OD values of the treatments at 90°C are significantly smaller than those of the other temperature, that is, when the medium is heated to 90-100°C, the number of miscellaneous bacteria is the least, and the rapid proliferation of miscellaneous bacteria is effectively suppressed. After further verification, it is determined that the pasteurization process is: heating the fermentation solution to 90~100°C and then directly cooling it down.

### 3. Selection of culture temperature

In this example, the inoculation amount is 10~50 million/mL (the amount of target bacteria is not less than 1~5 billion/mL to achieve a hundredfold amplification), the bacterial strain is inoculated into the fermentation container 2, the fermentation temperature is controlled at 30~40°C, the stirring speed is 200~300 rpm, the ventilation volume is 2~3 vvm, and the fermentation is carried out. The culture cycle is about 14~18h.

Different strains have different optimal fermentation temperatures. Taking Bacillus subtilis as a representative strain for aerobic fermentation to investigate the changes in the OD value of the fermentation solution at different temperatures over time. The results are shown in Table 3 below. Table 3 shows the changes in the OD value of the fermentation solution of aerobic micro-ecology strains as a function of the culture temperature:

**Table 3**

| | culture temperature | 0h | 2h | 4h | 6h | 8h | 10h | 12h | 24h |
|---|---|---|---|---|---|---|---|---|---|
| aerobic fermentation | 30°C | 0.515 | 0.317 | 0.82 | 1.043 | 1.62 | 1.59 | 1.267 | 1.7 |
| | 35°C | 0.515 | 0.457 | 1.169 | 1.755 | 2.815 | 2.48 | 2.73 | 7.64 |
| | 40°C | 0.515 | 0.56 | 0.95 | 1.57 | 2.37 | 2.34 | 2.69 | 4.6 |

Conclusion: Bacillus subtilis can grow normally under the condition of 30~40°C.

### 4. Selection of fermentation temperature

The medium solution (first liquid) is pasteurized and cooled by air cooling, which takes a long time and is basically unable to avoid the introduction of foreign miscellaneous bacteria. The longer the cooling time, the higher the probability of contamination by foreign miscellaneous bacteria.

Referring to above Table 3, Bacillus subtilis can metabolize normally at 30~40°C. Therefore, inoculation can be considered above the optimal growth temperature range to shorten the cooling time and reduce the chance of contamination by miscellaneous bacteria. The experimental data of the optimal inoculation temperature are shown in Table 4 below. Table 4 shows the effect of the initial inoculation temperature of aerobic micro-ecology strain fermentation on the proliferation of the strains:

**Table 4**

| inoculation temperature | 0h | 2h | 4h | 6h | 8h | 10h | 12h | 14h | 16h | 18h |
|---|---|---|---|---|---|---|---|---|---|---|
| 40°C | 0.523 | 0.544 | 0.892 | 1.438 | 1.962 | 2.327 | 2.574 | 3.968 | 4.332 | 4.471 |
| 42°C | 0.531 | 0.562 | 0.867 | 1.321 | 1.872 | 2.449 | 2.626 | 4.086 | 5.057 | 5.367 |
| 44°C | 0.525 | 0.515 | 0.529 | 0.654 | 0.863 | 1.092 | 1.711 | 2.716 | 3.513 | 3.724 |
| 46°C | 0.533 | 0.529 | 0.551 | 0.597 | 0.717 | 0.963 | 1.349 | 1.679 | 2.157 | 2.391 |
| 48°C | 0.520 | 0.517 | 0.542 | 0.578 | 0.672 | 0.867 | 1.067 | 1.460 | 1.716 | 2.037 |

Conclusion: When the inoculation temperature of the medium solution (first liquid) exceeds 42°C, the lag phase of Bacillus subtilis is significantly prolonged, resulting in the inability to increase the bacterial amount of fermentation or extend the fermentation cycle. Therefore, taking all factors into consideration, Bacillus subtilis microbial inoculant can be inoculated when the medium temperature does not exceed 42°C.

### 5. Selection of aeration devices

The smaller the bubbles in the fermentation container, the more complete the interface for oxygen supply, the greater the oxygen exchange, and the more vigorous the microbial growth. In this apparatus, due to the limitation of apparatus investment and space, the size of bubbles cannot be further reduced by stirring, so the size of bubbles introduced into the apparatus directly affects the supply of oxygen in the fermentation container.

The evaluation was performed using commonly used aeration apparatuses on the market. The results are shown in Table 5 below. Table 5 shows the effect of the aperture size of the aerobic micro-ecology strains aeration unit on the proliferation of strains:

**Table 5**

| aeration device | aperture size | ventilation volume | bacterial amount in fermentation |
|---|---|---|---|
| sand core aeration pipe | 50µm ~330µm | 2 vvm ~3vvm | 4.31 billion |
| aeration head | 50µm ~100µm | 2 vvm ~3vvm | 5.07 billion |
| microporous aeration rod | 5µm ~10µm | 2 vvm ~3vvm | 5.95 billion |

Conclusion: the smaller the aperture size of the aeration device, the better the aeration effect. In order to facilitate purchase and replacement, microporous aeration rods with an aperture size of 5~10µm are directly selected.

### 6. Selection of ventilation volume

Conventional aerobic fermentation generally adopts a combination of high-speed stirring and ventilation to provide sufficient oxygen for the strains. Here, the ventilation volume of 0.8~1.2vvm can meet the needs of aerobic fermentation strains.

Compared with the conventional fermentation system, the present disclosure greatly reduces the cost of the apparatus, adopts conventional magnetic stirring combined with bottom ventilation to supply air, and selects the stirring speed and ventilation volume with appropriate values to ensure the supply of oxygen. The specific data are shown in Table 6 below. Table 6 shows the effect of aerobic micro-ecology strains fermentation ventilation volume on the bacterial amount in fermentation:

**Table 6**

| stirring speed | ventilation volume | content of viable bacteria |
|---|---|---|
| 300~400rpm | 1.5vvm | 1.35 billion |
| | 2vvm | 4.61 billion |
| | 2.5vvm | 5.68 billion |
| | 3vvm | 5.73 billion |
| 200~300rpm | 1.5vvm | 2.04 billion |
| | 2vvm | 3.85 billion |
| | 2.5vvm | 5.97 billion |
| | 3vvm | 5.49 billion |
| 100~200rpm | 1.5vvm | 1.05 billion |
| | 2vvm | 4.02 billion |
| | 2.5vvm | 4.67 billion |
| | 3vvm | 5.06 billion |

Conclusion: the rotation speed is selected as 200~300rpm and the ventilation volume of 2~3vvm is sufficient for the fermentation of aerobic bacteria (Bacillus subtilis).

### 7. Completion of the fermentation

After 14~16h of fermentation, the pH value of the fermentation solution is about 6.0, and the viable bacteria content is not less than 5 billion/mL, then the fermentation can be stopped. The determination of the end of the fermentation can be determined by measuring the growth curve of viable bacteria. The specific data are shown in Table 7 below. Table 7 shows the bacterial amount of the aerobic micro-ecology strain fermentation at different culture time:

**Table 7**

| | bacterial amount of the aerobic fermentation, billion/mL |
|---|---|
| 0h | 4.95E+06 |
| 2h | 5.02E+06 |
| 4h | 4.89E+06 |
| 6h | 6.03E+06 |
| 8h | 1.38E+07 |
| 10h | 6.89E+07 |
| 12h | 2.47E+08 |
| 14h | 5.30E+08 |
| 16h | 5.56E+08 |
| 18h | 4.68E+08 |

Conclusion: The endpoint of aerobic fermentation can be selected at 14~16h of culture.

### 8. Discharging and cleaning

After the fermentation is completed, the fermentation solution can be directly pumped into the microbial inoculant application system or be temporarily stored in the fermentation container 2. After the discharge is completed, the first electronic valve 11 and the second electronic valve 12 are turned on, and the first driving unit 10 is started, and the liquid in the liquid storage tank 8 can be used to clean the residue on the inner wall of the fermentation container 2. From there, it's ready to move on to the next fermentation cycle.

### Example of anaerobic micro-ecology strain fermentation

The directed micro-ecology fermentation device provided in the embodiments of the present disclosure can implement both aerobic micro-ecology strain fermentation and anaerobic micro-ecology fermentation. Compared with the aerobic micro-ecology strain fermentation, the anaerobic micro-ecology fermentation only lacks ventilation during culture and uses intermittent stirring.

### 1. Determining the optimal pH value of the medium

First, a suitable medium is added into the fermentation container 2, and the initial pH value of the first liquid is determined to be about 6.8-7.0. The initial pH value of the medium directly affects the pH value of the medium after pasteurization, which in turn has a direct impact on the fermentation of the strains. Based on the relatively mature fermentation medium, Lactobacillus plantarum is used as the representative strain for anaerobic fermentation, and the selection of the most suitable initial pH value is shown in Table 8 below. Table 8 shows the effect of the initial pH value of anaerobic strain fermentation on the bacterial amount of fermentation:

**Table 8**

| fermentation mode | initial pH value | pH value after sterilization | bacterial amount |
|---|---|---|---|
| | 7.40 | 7.11 | 2.53 billion |
| | 7.20 | 6.86 | 2.84 billion |
| anaerobic fermentation | 7.00 | 6.70 | 5.29 billion |
| | 6.80 | 6.57 | 4.86 billion |
| | 6.60 | 6.37 | 4.41 billion |
| | 6.40 | 6.18 | 3.67 billion |

Conclusion: the most suitable initial pH value for anaerobic fermentation (of Lactobacillus plantarum) is 6.8-7.0.

### 2. Determining the optimal sterilization temperature

The second liquid is heated by the heater 5-2, and the second liquid heats the first liquid (medium) in the fermentation container 2, and the medium is pasteurized (heating power 5~7kw). Under a stirring condition, it is gradually heated from room temperature to 90~100°C, and the heating time is about 2~3h; then it is cooled by air, and the temperature is gradually reduced to 30~40°C, and the cooling time is about 1.5~3h (related to the room temperature).

The selection of pasteurization conditions is the same as that of the aerobic fermentation.

### 3. Selection of culture temperature

In this example, the inoculation amount is 10~50 million/mL, the strains are inoculated into the fermentation system, the fermentation temperature is controlled at 30~40°C. The fermentation is carried out under intermittent stirring and non-ventilated conditions. The culture cycle is about 14~18h.

Different strains have different most suitable propagation temperatures. Taking Lactobacillus plantarum as a representative strain for anaerobic fermentation to investigate the changes in the OD values of the fermentation solution at different temperatures. The results are shown in Table 9 below. Table 9 shows the changes in the OD values of the fermentation bacteria solutions of anaerobic strains as a function of the culture temperature:

**Table 9**

| | culture temperature | 0h | 2h | 4h | 6h | 8h | 10h | 12h | 24h |
|---|---|---|---|---|---|---|---|---|---|
| anaerobic fermentation | 30°C | 0.081 | 0.13 | 0.165 | 0.464 | 1.43 | 3.895 | 6.08 | 10.73 |
| | 35°C | 0.102 | 0.163 | 0.336 | 1.802 | 4.37 | 6.21 | 6.91 | 10.56 |
| | 40°C | 0.121 | 0.195 | 0.343 | 1.702 | 3.985 | 4.29 | 5.05 | 6.48 |

Conclusion: Lactobacillus plantarum can grow normally under the condition of 30-40°C.

### 4. Selection of stirring mode

Proper stirring helps to mix nutrients during anaerobic fermentation, but excessive stirring may introduce too much oxygen, thereby inhibiting the proliferation of the anaerobic bacteria. Therefore, it is necessary to select suitable stirring culture conditions to obtain higher bacterial amount in anaerobic fermentation.

The treatments of no stirring at all, continuous stirring and 3h-stirring-1min (stirring for 1 minute every 3 hours) were set respectively. The results are shown in Table 10 below. Table 10 shows the effect of anaerobic strain fermentation stirring mode on the proliferation of the strains:

**Table 10**

| stirring mode | OD values of the fermentation solution | content of viable bacteria |
|---|---|---|
| no stirring | 5.73 | 3.59 billion |
| continuous stirring | 6.09 | 4.38 billion |
| 3h-stirring-1min | 7.32 | 5.82 billion |

Conclusion: the mode of stirring for 1 minute every 3 hours is more conducive to the fermentation of the anaerobic bacteria.

### 5. Completion of the fermentation

At the end of the fermentation, the pH value of the fermentation solution is about 4.0, and the content of viable bacterial is not less than 5 billion/mL, then the fermentation can be terminated.

The determination of the end of the fermentation can be determined by measuring the growth curve of viable bacteria. The specific data are shown in Table 11 below. Table 11 shows the effect of the anaerobic strains fermentation time on the bacterial amount of fermentation:

**Table 11**

| | bacterial amount of the anaerobic fermentation, billion/mL |
|---|---|
| 0h | 1.08E+06 |
| 2h | 1.22E+06 |
| 4h | 1.99E+06 |
| 6h | 9.80E+07 |
| 8h | 3.25E+07 |
| 10h | 4.53E+08 |
| 12h | 5.67E+08 |
| 14h | 5.13E+07 |
| 16h | 5.37E+08 |
| 18h | 3.98E+08 |

Conclusion: anaerobic fermentation is completed in 12-18 hours.

### 6. Discharging and cleaning

After the fermentation is completed, the fermentation solution can be directly pumped into the microbial inoculant application system or be temporarily stored in the fermentation container 2. After the discharge is completed, the first electronic valve 11 and the second electronic valve 12 are turned on, and the first driving unit 10 is started, and the liquid in the liquid storage tank 8 can be used to clean the residue on the inner wall of the fermentation container 2. From there, it's ready to move on to the next fermentation cycle.

### Example of fermentation and enrichment of micro-ecology strains of polyglutamic acid and rhamnolipids

The directed micro-ecology fermentation device provided in the embodiments of the present disclosure can not only implement aerobic and anaerobic micro-ecology fermentations, but also support the enrichment of microbial metabolites.

### 1. Selection of bacterial strains and medium

Bacillus subtilis is selected as the high yield polyglutamic acid strain, which is isolated from soybean meal residue, and it is dried by spray after fermentation to obtain 100 billion bacterial powder. The selected enrichment medium for polyglutamic acid is: glucose 40 g/L, yeast powder 6 g/L, sodium glutamate 50 g/L, ammonium chloride 4 g/L, dipotassium hydrogen phosphate 7 g/L, magnesium sulfate heptahydrate 2 g/L, manganese sulfate monohydrate 0.5 g/L. The pH value of the polyglutamic acid enrichment medium is adjusted to 7.0.

Pseudomonas aeruginosa is selected as the high-yield rhamnolipids strain, and the strain is obtained from Northwestern University. The selected enrichment medium for rhamnolipids is: sodium nitrate 6 g/L, potassium chloride 0.2 g/L, magnesium sulfate 0.5 g/L, disodium hydrogen phosphate 5.13 g/L, sodium dihydrogen phosphate 0.76 g/L, glycerol 2 mL/L, olive oil 30 mL/L, and trace elements 0.2 mL/L. The pH value of the rhamnolipids enrichment medium is adjusted to 7.0 using sodium hydroxide (NaOH). Here, the formula for the trace elements (1 L) is as follows: manganese sulfate 39.9 mg, zinc sulfate 42.8 mg, and ammonium molybdate 34.7 mg.

### 2. Selection of culture conditions

This example involves two strains of aerobic bacteria, therefore, their cultivation conditions are mainly based on the aerobic micro-ecology fermentation example.

For Bacillus subtilis, the culture conditions are changed as follows: heat the fermentation solution to 90~100°C, directly cool it down, control the fermentation temperature at 37°C, the stirring speed is controlled at 200~300 rpm, and the ventilation volume is controlled at 2.5 vvm for fermentation, with a culture period of about 20 hours. Choose an initial inoculation volume of 50 million CFU/mL. Here, CFU stands for Colony Forming Units. After culture, take 500 mL of sample for retention.

For Pseudomonas aeruginosa, the culture conditions are changed as follows: heat the fermentation solution to 90~100°C, directly cool it down, the fermentation temperature is controlled at 30°C, the stirring speed is controlled at 200~300 rpm, and the ventilation volume is controlled at 2.5 vvm for fermentation, with a culture period of about 40 hours. Choose an initial inoculation volume of 20 million CFU/mL. After culture, take 500 mL of sample for retention.

### 3. Discharging and cleaning

After the fermentation is completed, the fermentation solution can be directly pumped into the microbial inoculant application system or be temporarily stored in the fermentation container 2. After the discharge is completed, the first electronic valve 11 and the second electronic valve 12 are turned on, and the first driving unit 10 is started, and the liquid in the liquid storage tank 8 can be used to clean the residue on the inner wall of the fermentation container 2. From there, it's ready to move on to the next fermentation cycle.

### 4. Preliminary purification and determination of polyglutamic acid in fermentation broth

Polyglutamic acid (γ-PGA) solution can react with sodium hydroxide solution of hexadecyltrimethylammonium bromide (CTAB) to form a suspension. The turbidity of such suspension can be reflected by measuring the absorbance. By preparing different concentrations of γ-PGA standard solutions and measuring the absorbance values, standard curves at different wavelengths can be drawn to establish a linear relationship between concentration and absorbance values, thereby the content of γ-PGA in fermentation broth can be quickly and accurately determined.

1). Preliminary purification

The polyglutamic acid fermentation broth is centrifuged at 4800 r/min for 25 minutes to remove bacterial cells. The supernatant is collected and three times the volume of anhydrous ethanol is added thereto. The mixture is left to stand overnight at 4°C and then centrifuged at 8000 r/min for 15 minutes to obtain a precipitate. The precipitate is dissolved in distilled water of the same volume as the fermentation broth to obtain a sample solution. The sample solution is diluted four times before measurement.

### 2). Preparation of CTAB solution

Prepare a 2% sodium hydroxide (NaOH) solution and use it as a solvent to add CTAB. After CTAB dissolves (can be heated appropriately to accelerate dissolution), prepare a 25 g/L CTAB test solution.

### 3). CTAB turbidity method

Take 2 mL of standard solution or sample solution into a test tube, accurately add 2 mL of CTAB test solution, start timing when adding, fully shake, try to avoid foam generated by the reaction solution during the process, stand for nearly 3 minutes, then pour the reaction solution into a cuvette, and measure the absorbance at the wavelength of 250 nm at 3 minutes.

### 4). Preparation and standard curve of γ-pga standard solution

Accurately weigh 3 g, 2.5 g, 2 g, 1.5 g, 1 g, and 0.5 g of γ-polyglutamic acid standard samples into clean beakers, respectively, add an appropriate amount of distilled water to dissolve, and then dilute to 100 mL in a volumetric flask. Shake well to obtain 30 g/L, 25 g/L, 20 g/L, 15 g/L, 10 g/L, and 5 g/L of γ-PGA standard samples, respectively.

After calculation (as shown in the table below), the standard curve is y = 0.0357x (R² = 0.9931), which has a good linear fit and can be used for quantitative analysis. The absorbance value of the fermented sample diluted four times is 0.63, and the final calculated concentration of γ-PGA in the sample is 70 g/L.

| concentration of γ-PGA X (g/L) | 5 | 10 | 15 | 20 | 25 | 30 |
|---|---|---|---|---|---|---|
| absorbance value Y | 0.157 | 0.364 | 0.58 | 0.719 | 0.9 | 1.037 |
| standard curve | y = 0.0357x (R² = 0.9931) | | | | | |
| absorbance value of the sample | 0.63 | | | | | |
| concentration of γ-PGA in the sample (g/L) | concentration of γ-PGA in the sample (g/L) = (0.63/0.0357)*4 = 70.5 | | | | | |

### 5. Determination of rhamnolipids in fermentation broth

The molecular structure of the surfactant glycolipid contains pentose. Under the action of hot concentrated sulfuric acid, dehydration occurs to form sugar aldehydes compounds. These sugar aldehydes compounds react with phenolic compounds (3,5-dihydroxytoluene, orcinol) to condense and produce colored substances. The maximum absorption peak is observed at visible wavelength (620 nm), and the corresponding glycolipid concentration is determined on the standard curve for quantitative analysis of biological surfactants.

### 1). Preparation of standard solution

Weigh 8.6 mg of dried and constant weight rhamnose reference sample, dissolve it in water, and dilute to a volume of 100 mL in a volumetric flask to prepare a standard solution of 0.086 mg/mL.

### 2). Preparation of anthrone reagent

Dissolve 0.20 g of anthrone in 100 mL of 85% sulfuric acid.

### 3). Drawing of standard curves

Precisely aspirate 1 mL, 2 mL, 3 mL, 4 mL, 5 mL, 6 mL, 7 mL, 8 mL, and 9 mL of standard solution into a 10 mL volumetric flask, add distilled water to the volume and shake well. Then aspirate 1 mL of each concentration of rhamnose solution and add 4 mL of anthrone test solution. Add 4 mL of 0.2% sulfuric acid anthrone reagent to an ice water bath, then mix well and immerse in a boiling water bath. After reboiling from the water bath for 15 minutes, take it out and cool it with tap water. After leaving at room temperature for 20 minutes, replace the standard solution with distilled water as a control and measure the absorbance at a wavelength of 620 nm.

Using the concentration of rhamnolipids as the horizontal axis and the absorbance value as the vertical axis, regression analysis was performed, and the regression equation is shown in the following table:

| | | | | | | |
|---|---|---|---|---|---|---|
| concentration of rhamnolipids X (g/L) | 0.000 | 0.0054 | 0.0107 | 0.0215 | 0.043 | 0.086 |
| absorbance value Y | 0.265 | 0.285 | 0.306 | 0.348 | 0.437 | 0.591 |
| standard curve | y = 3.8157x + 0.2661 (R² = 0.9992) | | | | | |

### 4). Determination of rhamnolipids in fermentation broth

Accurately aspirate 1 mL of diluted hundredfold fermentation broth and place it in a test tube. Add 4 mL of 0.2% sulfuric acid anthrone reagent to an ice water bath, then mix well and immerse in a boiling water bath. After 15 minutes, take it out and cool it with tap water, and leave it at room temperature for 20 minutes. Use uninoculated fermentation broth as a blank control, measure the absorbance at a wavelength of 620 nm and record the absorbance value A as 0.416. The yield of rhamnolipids (g/L) = (A-0.2661)/3.8157 × 3 × n (n is the dilution factor). Therefore, the final content of rhamnolipids in the fermentation broth is calculated to be 11.8 g/L.

According to an embodiment of the present disclosure, the method flow according to the embodiments of the present disclosure can be implemented as a computer software program. For example, an embodiment of the present disclosure includes a computer program product, which includes a computer program carried on a computer readable storage medium, and the computer program contains program codes for executing the method shown in the flowchart. According to embodiments of the present disclosure, the electronic apparatus, apparatus, device, modules, units, etc. described above may be implemented by a computer program module.

The present disclosure also provides a computer readable storage medium, which may be included in the apparatus/device/system described in the above embodiments; or may exist independently without being assembled into the apparatus/device/system. The computer readable storage medium carries one or more programs. When the one or more programs are executed, the method according to the embodiments of the present disclosure is implemented.

According to embodiments of the present disclosure, the computer readable storage medium may be a non-volatile computer readable storage medium, for example, may include but is not limited to: a portable computer disk, a hard disk, a random access memory (RAM), a read-only memory (ROM), an erasable programmable read-only memory (EPROM or flash memory), a portable compact disk read-only memory (CD-ROM), an optical storage device, a magnetic storage device, or any suitable combination of the above. In the present disclosure, a computer readable storage medium may be any tangible medium that contains or stores a program that can be used by or in conjunction with an instruction execution system, apparatus, or device.

The flowcharts and block diagrams in the accompanying drawings illustrate possible implementation architectures, functions, and operations of systems, methods, and computer program products according to various embodiments of the present disclosure. In this regard, each block in the flowchart or block diagram may represent a module, a program segment, or a portion of code, which may contain one or more executable instructions for implementing specified logical functions. It should also be noted that, in some alternative implementations, the functions noted in the block may occur out of the order noted in the figures. For example, two blocks shown in succession may actually be executed substantially in parallel, or they may sometimes be executed in the reverse order, depending on the functionality involved. It should also be noted that each block in the block diagram or flowchart, and combinations of blocks in the block diagram or flowchart, can be implemented by a dedicated hardware-based system that performs the specified function or operation, or can be implemented by a combination of dedicated hardware and computer instructions.

Those skilled in the art will appreciate that the features described in the embodiments of the present disclosure may be combined or/and coupled in various ways, even if such combinations or couplings are not explicitly described in the present disclosure. In particular, various combinations and/or couplings of features described in the embodiments of the present disclosure may be made without departing from the spirit and teaching of the present disclosure. All such combinations and/or couplings fall within the scope of the present disclosure.

The embodiments of the present disclosure have been described above. However, these embodiments are for illustrative purposes only and are not intended to limit the scope of the present disclosure. Although the embodiments are described above separately, this does not mean that measures in the various embodiments cannot be advantageously used in combination. Without departing from the scope of the present disclosure, those skilled in the art may make various substitutions and modifications, all of which should fall within the scope of the present disclosure.

## Claims

1. A directed micro-ecology fermentation device, comprising:
a housing (1);
a fermentation container (2), which is provided in the housing (1) and configured to provide a place for fermentation of a microbial inoculant;
a liquid injection port (3), which is provided on the housing (1), communicated with the fermentation container (2) and configured to inject a first liquid into the fermentation container (2);
a temperature control unit (5), which is provided in the housing (1) and configured to heat the first liquid in the fermentation container (2) and incompletely sterilize the first liquid;
a microbial inoculant adding unit (4), which is provided on the housing (1), communicated with the fermentation container (2), and configured to add the microbial inoculant into the fermentation container (2);
a stirrer (6), which is provided in the fermentation container (2) and configured to stir the first liquid in the fermentation container (2) to promote fermentation, wherein the temperature control unit (5) is configured to control a temperature of the first liquid in the fermentation container (2); and
a discharge port (7), which is provided on the housing (1), communicated with the fermentation container (2) and configured to discharge fermented materials in the culture container (2).

2. The directed micro-ecology fermentation device according to claim 1, further comprising:
a liquid storage tank (8), which is provided in the housing (1), communicated with the liquid injection port (3) and the fermentation container (2), and configured to store the first liquid for fermentation and/or a liquid for cleaning residue on an inner wall of the fermentation container (2).

3. The directed micro-ecology fermentation device according to claim 2, further comprising:
an ejecting head (9), which is provided inside the fermentation container (2), communicated with the liquid injection port (3) and the liquid storage tank (8), and configured to clean residue on the inner wall of the fermentation container (2); and
a first driving unit (10), which is provided between the ejecting head (9) and the liquid storage tank (8), and configured to drive a liquid in the liquid storage tank (8) to clean residue on the inner wall of the fermentation container (2).

4. The directed micro-ecology fermentation device according to claim 1, further comprising:
an aeration unit (17), which is provided in the housing (1), communicated with the fermentation container (2), and configured to introduce air into the fermentation container (2) so as to allow an aerobic fermentation for the microbial inoculant.

5. The directed micro-ecology fermentation device according to claim 4, further comprising:
an air filter (18), which is provided between the aeration unit (17) and the fermentation container (2) and configured to filter impurities and water vapor in air.

6. The directed micro-ecology fermentation device according to claim 1, wherein the temperature control unit (5) comprises:
a liquid jacket (5-1), which is provided inside a shell of the fermentation container (2) and configured to heat or cool the first liquid in the fermentation container (2) by using a second liquid in the liquid jacket (5-1) by means of heat transfer, wherein the second liquid in the liquid jacket (5-1) is isolated from the first liquid in the fermentation container (2);
a heater (5-2), which is configured to heat the second liquid in the liquid jacket (5-1) and incompletely sterilize the first liquid, wherein a liquid outlet of the heater (5-2) is in communication with a liquid inlet of the liquid jacket (5-1), and a liquid inlet of the heater (5-2) is in communication with a liquid outlet of the liquid jacket (5-1);
a cooler (5-3), which is communicated in parallel with the heater (5-2) and configured to cool the second liquid in the liquid jacket (5-1); and
a second driving unit (5-4), which is communicated in series with the heater (5-2) and configured to drive the second liquid in the liquid jacket (5-1) to circulate.

7. The directed micro-ecology fermentation device according to claim 6, wherein the microbial inoculant adding unit (4) comprises:
a microbial inoculant box (4-1), which is provided in the housing (1) and configured to store the microbial inoculants;
a microbial inoculant delivery pipeline (4-2), which is provided between the microbial inoculant box (4-1) and the fermentation container (2) and configured to deliver the microbial inoculants stored in the microbial inoculant box (4-1) to the fermentation container (2), wherein the microbial inoculant delivery pipeline (4-2) is made of a heat-insulating material; and
an electronic switch (4-3), which is provided between the microbial inoculant box (4-1) and the microbial inoculant delivery pipeline (4-2) and configured to deliver a predetermined amount of the microbial inoculants to the fermentation container (2) through the microbial inoculant delivery pipeline (4-2) under a control of a signal.

8. The directed micro-ecology fermentation device according to claim 1, wherein a blade (6-1) of the stirrer (6) is provided at an angle and rotates in a counter-clockwise direction, a front side (b) of the blade (6-1) is tilted, a rear side (a) of the blade (6-1) is drooped, and a distance between the front side (b) and a bottom of the fermentation container (2) is greater than a distance between the rear side (a) and the bottom of the fermentation container (2).

9. A fermentation method for the directed micro-ecology fermentation device according to any one of claims 1 to 8, the fermentation method comprising:
controlling an electronic valve to inject a predetermined amount of the first liquid into the fermentation container;
sterilizing the injected first liquid by means of a non-complete sterilization manner;
adding a microbial inoculant into the fermentation container according to a liquid level of the first liquid after non-complete sterilization; and
performing, by adopting set conditions, fermentation on the first liquid to which the microbial inoculant is added according to a type of the microbial inoculant.

10. The fermentation method according to claim 9, wherein, after the step of performing, by adopting set conditions, fermentation on the first liquid to which the microbial inoculant is added according to the type of the microbial inoculant, the fermentation method further comprising:
cleaning residue on an inner wall of the fermentation container with a liquid pre-stored in the directed micro-ecology fermentation device.

11. The fermentation method according to claim 9, wherein, before the step of adding a microbial inoculant into the fermentation container according to a liquid level of the first liquid after non-complete sterilization, the fermentation method further comprising:
adjusting a pH value of the first liquid according to the type of the microbial inoculant to be added.

12. The fermentation method according to claim 9, wherein, after the step of performing, by adopting set conditions, fermentation on the first liquid to which the microbial inoculant is added according to the type of the microbial inoculant, the fermentation method further comprising:
determining an end of the fermentation according to a curve showing an OD value of the first liquid changing with time.

13. The fermentation method according to claim 9, wherein, the step of sterilizing the injected first liquid by means of a non-complete sterilization manner comprises:
controlling a heater to heat a second liquid in a liquid jacket, wherein the liquid jacket is provided inside a shell of the fermentation container, and the second liquid is isolated from the first liquid;
heating the first liquid in the fermentation container with the second liquid;
cooling the second liquid in the liquid jacket with a cooler; and
cooling the first liquid in the fermentation container with the second liquid.

14. The fermentation method according to claim 13, wherein the second liquid includes at least one of oil, water and liquid gas.

15. The fermentation method according to claim 9, wherein, if the microbial inoculant is an aerobic microbial inoculant, the step of performing, by adopting set conditions, fermentation on the first liquid to which the microbial inoculant is added according to the type of the microbial inoculant comprises:
controlling a temperature of the first liquid to which the microbial inoculant is added by the temperature control unit;
supplying air to the first liquid to which the microbial inoculant is added with an aeration unit at a predetermined ventilation volume; and
stirring the first liquid to which the microbial inoculant is added with a stirrer at a predetermined rotation speed.

16. The fermentation method according to claim 15, wherein, before the step of supplying air to the first liquid to which the microbial inoculant is added with an aeration unit at a predetermined ventilation volume, the step of performing, by adopting set conditions, fermentation on the first liquid to which the microbial inoculant is added according to the type of the microbial inoculant further comprises:
filtering out impurities and moisture in air by an air filter.

17. The fermentation method according to claim 15, wherein,
the step of supplying air to the first liquid to which the microbial inoculant is added with an aeration unit at a predetermined ventilation volume comprises:
supplying air to the first liquid to which the microbial inoculant is added with the aeration unit at a ventilation volume of 2~3 vvm, and
the step of stirring the first liquid to which the microbial inoculant is added with a stirrer at a predetermined rotation speed comprises:
stirring the first liquid to which the microbial inoculant is added with the stirrer at a rotation speed of 200~300 rpm.

18. The fermentation method according to claim 9, wherein, if the microbial inoculant is an anaerobic microbial inoculant, the step of performing, by adopting set conditions, fermentation on the first liquid to which the microbial inoculant is added according to the type of the microbial inoculant comprises:
controlling a temperature of the first liquid to which the microbial inoculant is added by the temperature control unit; and
periodically stirring the first liquid to which the microbial inoculant is added with the stirrer.

19. The fermentation method according to claim 18, wherein, the step of periodically stirring the first liquid to which the microbial inoculant is added with the stirrer comprises:
stirring the first liquid for one minute every three hours with the stirrer.

20. A computer readable storage medium having executable instructions stored thereon, wherein the instructions, when executed by a processor, implement the method according to any one of claims 9 to 19.
